# EUROPEAN PATENT APPLICATION

(11) **EP 4 374 866 A1**
(43) Date of publication of application: **29.05.2024**
(21) Application number: 22846162.0
(22) Date of filing: 18.07.2022
(51) Int. Cl.: A61K 36/232, A61K 36/69, A61K 36/77, A61K 31/7016, A61P 17/02, A61P 19/02, A61P 29/00, A61P 17/06, A61P 31/04, A61K 8/9789

(54) **COMPOSITION FOR PREVENTING, AMELIORATING OR TREATING SKIN WOUNDS OR INFLAMMATION**

(30) Priority: 19.07.2021 KR 20210093882; 15.07.2022 KR 20220087251
(71) Applicant: Medihelpline Co., Ltd., Seoul 04537 (KR)
(72) Inventor: PARK, Ok Nam, Seoul, Seoul 06981 (KR)
(74) Representative: Frenkel, Matthias Alexander
(86) International application number: PCT/KR2022/010422
(87) International publication number: WO 2023/003292

(57) **Abstract**

The present invention relates to a composition for preventing, improving or treating skin wound or inflammation. According to the present invention, since the composition is effective in treating not only general wound and inflammation occurring on the skin, but also chronic wound such as diabetic foot ulcers and bedsores, and chronic inflammation such as atopic dermatitis and psoriasis, the composition may be effectively used to prevent, improve, or treat skin wound or inflammation.

## Description

### [Technical Field]

The present invention relates to a composition for preventing, improving or treating skin wound or inflammation.

### [Background Art]

The skin is an organ that is exposed to chemical, mechanical, and biological stimuli and functions as a barrier to protect the human body, and bioactive damage that commonly occurs on the skin generally occurs to be wounded on the epidermal layer or the dermal layer and is naturally healed. However, when the physiological functions of cells related to a wound healing process in hemostasis, inflammation, and regenerative stages are not smooth, it may not only worsen skin diseases but also accelerate aging, causing cosmetic and pathological problems in the skin.

The wound healing is generally a complex process involving three steps of inflammation, proliferation, and remodeling. The first step involves clotting to achieve hemostasis, replenishment of neutrophils to destroy bacteria and necrotic tissue, and then replenishment of macrophages. During the second step, angiogenesis occurs, and at this time, endothelial cells migrate to the wound site, and simultaneously, fibroblasts migrate to the wound site to help in producing granulation tissue. The formation of granulation tissue may cause re-epithelialization. During the final step, the levels of collagen production and destruction are equalized and the healed wound slowly changes to achieve maximum strength. The wound healing may become chronic when any one of the processes does not function properly in a timely manner to be delayed or impaired.

Chronic wound is defined as open wound in the skin that requires treatment for 8 weeks or more. Impaired healing processes may often be associated with diabetic complications, leading to serious outcomes including high limb amputation rate and even death. Approximately 15% of diabetic patients suffer from chronic wound that is not healed. Accordingly, the wound is an important issue not only for individuals but also for society, and the risk of secondary infection may be reduced only when the wound is treated early.

Effective wound healing requires highly organized integrated coordination through complex molecular biological events, including extra-cellular matrix (ECM) deposition and cell proliferation/migration. One of major factors associated with the development of chronic wound is damage to a cytokine secretion system secreted by local fibroblasts and inflammatory cells, which causes a decrease in angiogenesis.

The inflammatory response in the skin begins as a defense function when skin damage is caused by physical stimuli, chemicals, bacteria, etc., and involved with various immune cells and inflammation-inducing cytokines. Tumor necrosis factor-α (TNF-α), interleukin-1β (IL-1β), interleukin-6 (IL-6), and interleukin-8 (IL-8) are representative inflammation-inducing cytokines. In addition, activated macrophages produce excessively nitric oxide (NO) and prostaglandin E2 (PGE2) as well as inflammation-inducing cytokines to further activate the inflammatory process.

The inflammatory response of the skin is regulated by various complex genes, and cyclooxygenase-2 (COX-2) and inducible nitric oxide synthases (iNOS) are known as major genes. Among the genes, COX-2 is known to cause PGE2 production by inflammatory cytokines, cell growth factors, cancer cell promoting factors, and reactive oxygen species (ROS), and inflammatory skin diseases such as psoriasis, pyoderma, and lupus erythematosus. Accordingly, substances capable of inhibiting the expression or activity of these skin inflammation-inducing factors may be used as agents for improvement and treatment of skin inflammation.

In addition, as various harmful effects of synthetic raw materials used in cosmetics, for example, side effects such as skin irritation and flushing, become known, interest in natural raw materials that may be applied to cosmetics is gradually increasing. However, despite research to discover combinations and derived materials of plant-derived extracts that exhibit skin functionality so far, there have been very few cases where combinations or derived materials of plant-derived extracts showing satisfactory effects have been identified and applied to cosmetic compositions.

Accordingly, the present inventors completed the present invention by conducting research to discover materials that exhibits skin wound and inflammation treatment activity.

### [Disclosure]

### [Technical Problem]

One object of the present invention is to provide a pharmaceutical composition for preventing or treating skin wound or inflammation including an extract of at least one plant selected from the group consisting of Ligustici Rhizoma(Ligusticum tenuissimum Kitagawa), Polygala Root(Polygala tenuifolia Willdenow), and Longan Arillus(Dimocarpus longan Loureiro), or a compound represented by Chemical Formula 1 or Chemical Formula 2 below or a pharmaceutically acceptable salt thereof:

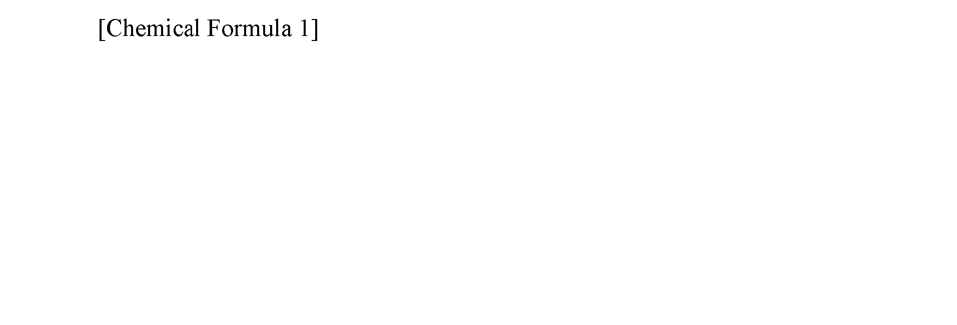

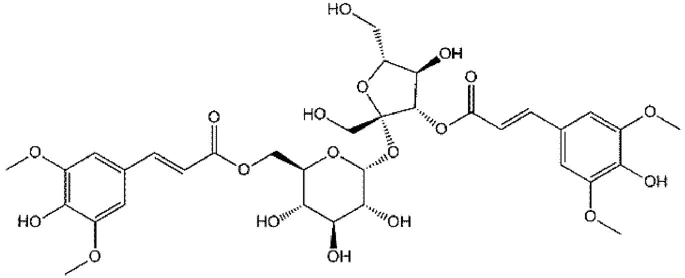

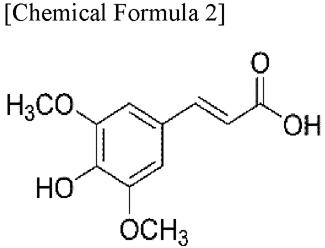

Another object of the present invention is to provide a cosmetic composition for preventing or improving skin wound or inflammation including an extract of at least one plant selected from the group consisting of Ligustici Rhizoma, Polygala Root, and Longan Arillus, or a compound represented by Chemical Formula 1 or Chemical Formula 2 below or a pharmaceutically acceptable salt thereof:

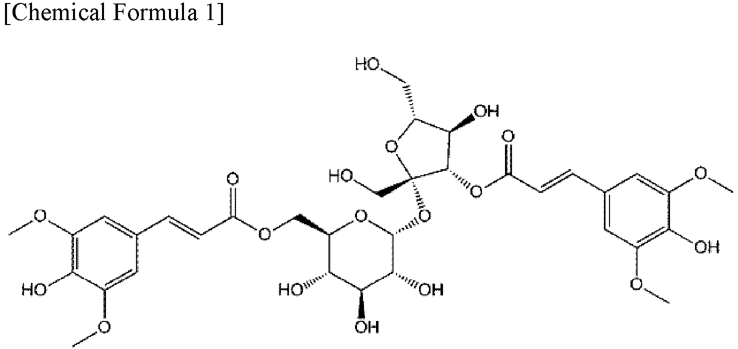

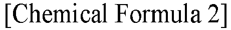

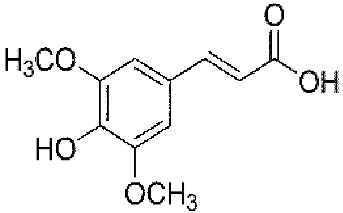

Yet another object of the present invention is to provide a food composition for preventing or improving skin wound or inflammation including an extract of at least one plant selected from the group consisting of Ligustici Rhizoma, Polygala Root, and Longan Arillus, or a compound represented by Chemical Formula 1 or Chemical Formula 2 below or a pharmaceutically acceptable salt thereof:

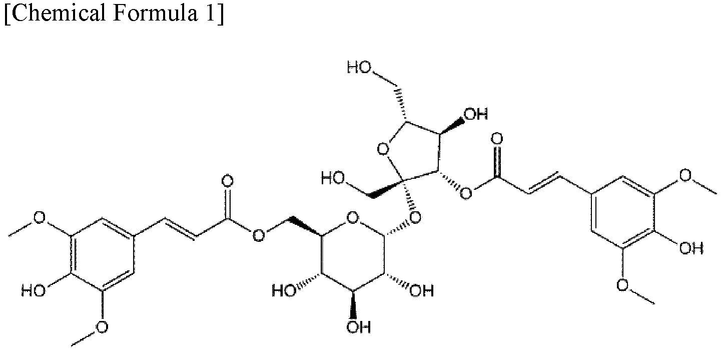

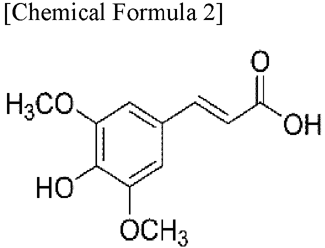

Yet another object of the present invention is to provide a method for treating skin wound or inflammation including administering the composition to a subject.

### [Technical Solution]

One aspect of the present invention provides a pharmaceutical composition for preventing or treating skin wound or inflammation including an extract of at least one plant selected from the group consisting of Ligustici Rhizoma, Polygala Root, and Longan Arillus, or a compound represented by Chemical Formula 1 or Chemical Formula 2 below or a pharmaceutically acceptable salt thereof:

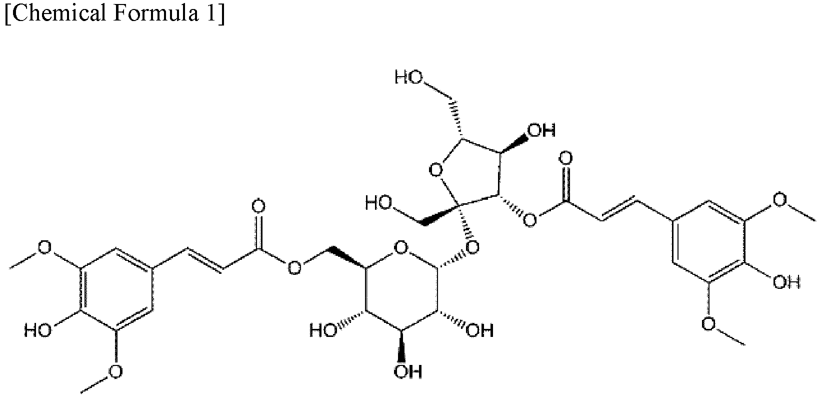

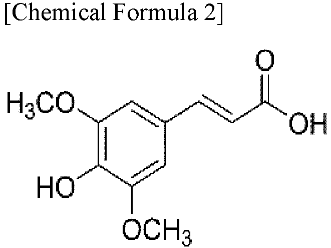

According to one embodiment of the present invention, the extract may be extracted with any one solvent selected from the group consisting of water, alcohols having 1 to 4 carbon atoms, and mixed solvents thereof.

According to one embodiment of the present invention, the pharmaceutical composition may be at least one formulation selected from the group consisting of creams, gels, patches, sprays, emulsions, ointments, emplastrum, lotions, liniment agents, paste agents, solvents, suspensions, packs, patches and cataplasma.

According to one embodiment of the present invention, the inflammation may be skin inflammation or arthritis.

According to one embodiment of the present invention, the skin inflammation may be any one disease selected from the group consisting of chronic skin inflammation, allergic skin inflammation, atopic dermatitis, and psoriasis.

According to one embodiment of the present invention, the skin wound may be diabetic foot ulcer or bedsore.

Another aspect of the present invention provides a cosmetic composition for preventing or improving skin wound or inflammation including an extract of at least one plant selected from the group consisting of Ligustici Rhizoma, Polygala Root, and Longan Arillus, or a compound represented by Chemical Formula 1 or Chemical Formula 2 below or a pharmaceutically acceptable salt thereof:

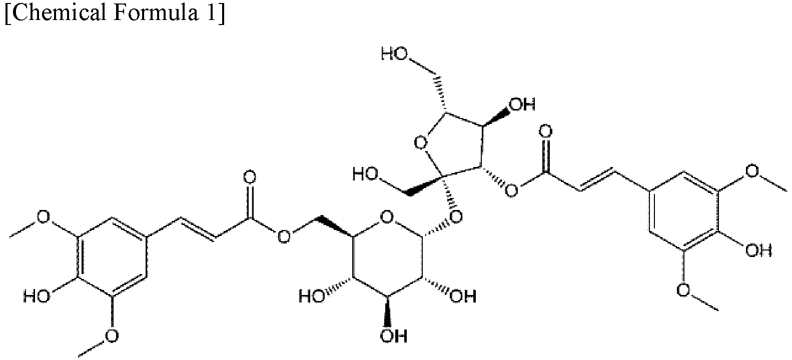

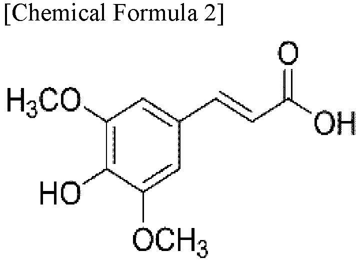

According to one embodiment of the present invention, the extract may be extracted with any one solvent selected from the group consisting of water, alcohols having 1 to 4 carbon atoms, and mixed solvents thereof.

According to one embodiment of the present invention, the cosmetic composition may be any one formulation selected from the group consisting of a solution, a suspension, an emulsion, a paste, a gel, a cream, a lotion, a powder, a soap, a surfactant-containing cleansing, oils, a powder foundation, an emulsion foundation, a wax foundation and a spray.

According to one embodiment of the present invention, the skin inflammation may be any one disease selected from the group consisting of chronic skin inflammation, allergic skin inflammation, atopic dermatitis, and psoriasis.

According to one embodiment of the present invention, the skin wound may be diabetic foot ulcer or bedsore.

Yet another aspect of the present invention provides a food composition for preventing or improving skin wound or inflammation including an extract of at least one plant selected from the group consisting of Ligustici Rhizoma, Polygala Root, and Longan Arillus, or a compound represented by Chemical Formula 1 or Chemical Formula 2 below or a pharmaceutically acceptable salt thereof:

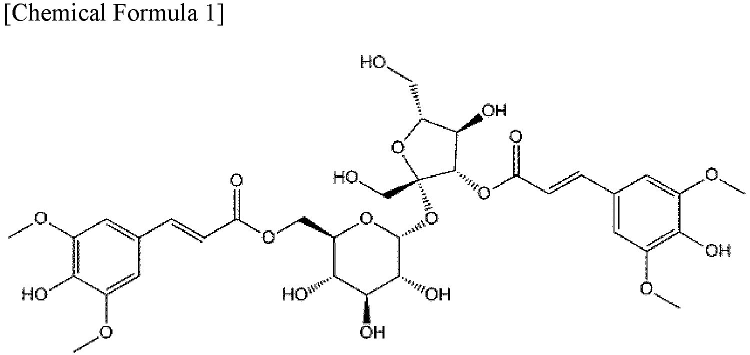

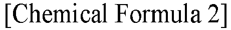

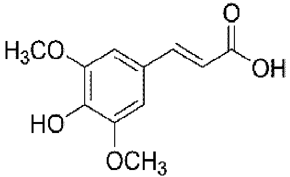

According to one embodiment of the present invention, the extract may be extracted with any one solvent selected from the group consisting of water, alcohols having 1 to 4 carbon atoms, and mixed solvents thereof.

According to one embodiment of the present invention, the skin inflammation may be any one disease selected from the group consisting of chronic skin inflammation, allergic skin inflammation, atopic dermatitis, and psoriasis.

According to one embodiment of the present invention, the skin wound may be diabetic foot ulcer or bedsore.

Yet another aspect of the present invention provides a method for treating skin wound or inflammation including administering the composition to a subject.

### [Advantageous Effects]

According to the present invention, since the composition for preventing, improving or treating skin wound or inflammation is effective in treating not only general wound and inflammation occurring on the skin, but also chronic wound such as diabetic foot ulcers and bedsores, and chronic inflammation such as atopic dermatitis and psoriasis, the composition can be effectively used to prevent, improve, or treat skin wound or inflammation.

### [Description of Drawings]

FIG. 1 is a diagram illustrating a result of confirming cell migration by a cell scratch test method in order to confirm a wound healing effect of Ligustici Rhizoma, Polygala Root and/or Longan Arillus, 3',6-disinapoyl sucrose, or sinapic acid.
FIG. 2 is a diagram illustrating a result of confirming the gene expression levels of TNF-α (A) and IL-6 (B) using real time RT-PCR in order to confirm a wound healing effect of Ligustici Rhizoma, Polygala Root and/or Longan Arillus, 3',6-disinapoyl sucrose, or sinapic acid.

### [Best Mode]

One aspect of the present invention provides a pharmaceutical composition for preventing or treating skin wound or inflammation including an extract of at least one plant selected from the group consisting of Ligustici Rhizoma, Polygala Root, and Longan Arillus, or a compound represented by Chemical Formula 1 or Chemical Formula 2 below or a pharmaceutically acceptable salt thereof:

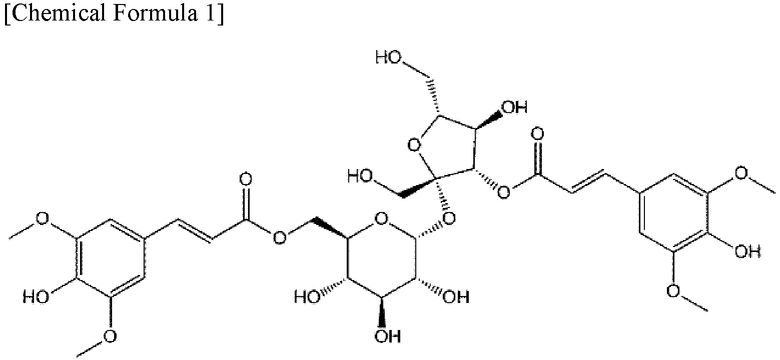

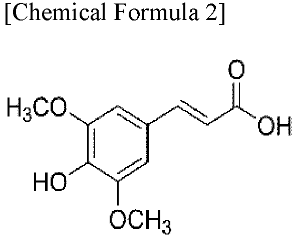

The compound represented by Chemical Formula 1 of the present invention may be 3',6-disinapoyl sucrose, and the compound represented by Chemical Formula 2 of the present invention may be sinapic acid, and these compounds may be extracted from Polygala Root, but are not limited thereto.

In the present invention, Ligustici Rhizoma, Polygala Root, and Longan Arillus may be sold and purchased commercially, or those collected or cultivated in nature may be used.

According to one embodiment of the present invention, the extract may be extracted with any one solvent selected from the group consisting of water, alcohols having 1 to 4 carbon atoms, and mixed solvents thereof.

The extract included in the composition according to the present invention may be obtained as follows. At least one plant selected from the group consisting of Ligustici Rhizoma, Polygala Root, and Longan Arillus is washed with water to remove foreign substances and then dried in the shade, and fully immersed by adding an appropriate amount of solvent. At this time, the plant may be used in its dried state as it is or crushed and used in a powder form. The extract may be extracted using general extraction solvents, preferably (a) anhydrous or hydrous lower alcohols having 1 to 4 carbon atoms (e.g., methanol, ethanol, propanol, butanol, n-propanol, iso-propanol and n-butanol, etc.), (b) mixed solvents of the lower alcohols and water, (c) acetone, (d) ethyl acetate, (e) chloroform, (f) 1,3-butylene glycol, (g) hexane, (h) diethyl ether, (i) butyl acetate (j) chloroform-methanol or (k) distilled water, and preferably any one solvent selected from the group consisting of water, alcohols having 1 to 4 carbon atoms, and mixed solvents of thereof. During extraction, the extract may be impregnated at room temperature or heated.

The pharmaceutical composition of the present invention may include a pharmaceutically acceptable carrier. The pharmaceutically acceptable carrier to be included in the pharmaceutical composition of the present invention is generally used in preparation of drugs, and includes lactose, dextrose, sucrose, sorbitol, mannitol, starch, acacia gum, calcium phosphate, alginate, gelatin, calcium silicate, microcrystalline cellulose, polyvinylpyrrolidone, cellulose, water, syrup, methylcellulose, methylhydroxybenzoate, propylhydroxybenzoate, talc, magnesium stearate, mineral oil, and the like, but is not limited thereto. The pharmaceutical composition of the present invention may further include a lubricant, a wetting agent, a sweetening agent, a flavoring agent, an emulsion, a suspension, a preservative, and the like, in addition to the ingredients. Suitable pharmaceutically acceptable carriers and formulations are described in detail in *the* Remington's Pharmaceutical Sciences (22th ed., 2013).

According to one embodiment of the present invention, the pharmaceutical composition may be administered together with one or more substances that exhibit the activity of preventing or treating skin wound or inflammation.

In addition, the pharmaceutical composition according to one embodiment of the present invention may be used alone or in combination with methods using surgery, hormone therapy, drug therapy, and/or biological response regulators, for the prevention or treatment of skin wound or inflammation.

The pharmaceutical composition of the present invention may include various bases and/or additives necessary and appropriate for the preparation of formulations thereof, and without departing from the range of reducing the effects thereof, may be prepared by further including known compounds, such as nonionic surfactants, silicone polymers, extenders, fragrances, preservatives, disinfectants, oxidation stabilizers, organic solvents, ionic or nonionic thickeners, softeners, antioxidants, free radical destroyers, opacifiers, stabilizers, emollients, silicone, α-hydroxy acid, anti-foaming agents, moisturizers, vitamins, insect repellents, flavorings, preservatives, surfactants, anti-inflammatory agents, substance P antagonists, fillers, polymers, propellants, alkalinizing or acidifying agents, or coloring agents.

A suitable dose of the pharmaceutical composition of the present invention may be variously prescribed by factors, such as a formulation method, an administration method, age, weight, and sex of a patient, a pathological condition, food, an administration time, an administration route, an excretion rate, and response susceptibility. The dose of the pharmaceutical composition of the present invention may be 0.001 to 1000 mg/kg for adults.

The pharmaceutical composition of the present invention may be administered orally or parenterally.

The pharmaceutical composition of the present invention may be administered in various formulations when administered orally, and may be administered in the form of solid preparations such as pills, powders, granules, tablets, or capsules, and may be administered in the form of various excipients, such as wetting agents, sweeteners, aromatics, preservatives, etc. Specifically, when the composition of the present invention is formulated in the form of powder, granule, tablet or capsule, the composition may further include appropriate carriers, excipients and diluents commonly used in its preparation. The carriers, excipients and diluents may be used with, for example, lactose, dextrose, sucrose, sorbitol, mannitol, xylitol, erythritol, maltitol, starch, gum acacia, alginate, gelatin, calcium phosphate, calcium silicate, cellulose, methyl cellulose, microcrystalline cellulose, polyvinyl pyrrolidone, water, methylhydroxybenzoate, propylhydroxybenzoate, talc, magnesium stearate and/or mineral oil, but are not limited thereto. In addition, the composition may be prepared by including diluents or excipients such as fillers, extenders, binders, wetting agents, disintegrants, and surfactants commonly used in formulation, and may further include lubricants such as magnesium stearate or talc in addition to the excipients.

The pharmaceutical composition of the present invention may be administered in various formulations when administered parenterally. Solid formulations include tablets, pills, powders, granules, capsules, etc., and liquid formulations correspond to suspensions, oral solutions, emulsions, syrups, etc., and may include various excipients, such as wetting agents, sweeteners, fragrances, and preservatives, in addition to commonly used simple diluents such as water, liquid, and paraffin. Specifically, the formulations for parenteral administration may include a sterile aqueous solution, a non-aqueous solution, a suspension, an emulsion, and a lyophilizing agent. As the non-aqueous solution and the suspension, propylene glycol, polyethylene glycol, vegetable oil such as olive oil, injectable ester such as ethyl oleate, and the like may be used. In addition, calcium or vitamin D3 may be added to improve the efficacy of therapeutic agents. These compositions may be presented in unit-dose (single-dose) or multi-dose (several-dose) containers, such as sealed ampoules and vials, and may be stored under freeze-dried conditions requiring only the addition of a sterile liquid carrier, such as injections, immediately before use. Immediate preparations and suspensions may be prepared from sterile powders, granules and tablets.

According to an embodiment of the present invention, the pharmaceutical composition may be at least one formulation selected from the group consisting of creams, gels, patches, sprays, emulsions, ointments, emplastrum, lotions, liniment agents, paste agents, solvents, suspensions, packs, patches and cataplasma.

In addition, the pharmaceutical composition of the present invention may be prepared as a dressing formulation including a drug layer containing an extract of at least one plant selected from the group consisting of Ligustici Rhizoma, Polygala Root, and Longan Arillus, or a compound represented by Chemical Formula 1 or Chemical Formula 2 or a pharmaceutically acceptable salt thereof as an active ingredient.

The drug layer may preferably have a thickness in the range of 10 to 200 µm.

The drug layer may further include substances known as pharmaceuticals in the related art in addition to an extract of at least one plant selected from the group consisting of Ligustici Rhizoma, Polygala Root, and Longan Arillus, or a compound represented by Chemical Formula 1 or Chemical Formula 2 or a pharmaceutically acceptable salt, but is not limited thereto, and substances that may directly or indirectly help wound healing, such as viscosity modifiers or wound healing aids. The substances may be appropriately selected by those skilled in the art depending on the purpose of the dressing agents. For example, for the purpose of a dressing agent used on wound sites, especially to prevent infection and proliferation of various bacteria, antibacterial agents may be further included. These antibacterial agents may include, for example, silver sulfadiazine, povidone iodine, iodine, iodide ion salt, pladiomycin sulfate, acrinol, chlorhexidine, benzalkonium chloride, benzethonium chloride, sodium fusidate, etc., but are not limited thereto.

In addition, the drug layer may further include known substances known to be effective in treating skin wound, and may further include a moisturizer and/or a wound healing aid, and the drug layer may be prepared by further including a solvent, an excipient, and a viscosity modifier. The solvent may be used without limitation as long as the solvent is a pharmaceutically acceptable solvent that may dissolve or disperse the selected drug, and for example, the solvent may be used with deionized water (DIW), ethanol, etc. Excipients such as glycerin may be used, and viscosity modifiers such as carboxymethyl cellulose, sodium alginate, and carrageenan may be used.

According to one embodiment of the present invention, the inflammation may be skin inflammation or arthritis.

According to one embodiment of the present invention, the skin inflammation may be any one disease selected from the group consisting of chronic skin inflammation, allergic skin inflammation, atopic dermatitis, and psoriasis.

According to one embodiment of the present invention, the skin wound may be diabetic foot ulcer or bedsore.

Another aspect of the present invention provides a cosmetic composition for preventing or improving skin wound or inflammation including an extract of at least one plant selected from the group consisting of Ligustici Rhizoma, Polygala Root, and Longan Arillus, or a compound represented by Chemical Formula 1 or Chemical Formula 2 below or a pharmaceutically acceptable salt thereof:

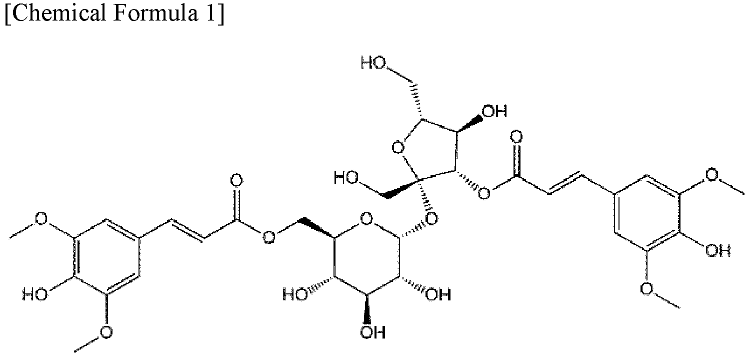

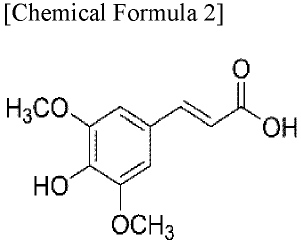

In order to avoid excessive complexity of the specification, the description of content that overlaps with the aforementioned content is omitted.

According to one embodiment of the present invention, the extract may be extracted with any one solvent selected from the group consisting of water, alcohols having 1 to 4 carbon atoms, and mixed solvents thereof.

The cosmetic composition of the present invention may further include adjuvants commonly used in the cosmetic field, such as fatty substances, organic solvents, solubilizers, thickeners, gelling agents, softeners, antioxidants, suspensions, stabilizers, foaming agents, fragrances, surfactants, water, ionic or nonionic emulsifiers, fillers, metal ions sequestering agents and chelating agents, preservatives, vitamins, blocking agents, humectants, essential oils, dyes, pigments, hydrophilic or lipophilic activators, lipid vesicles or any other ingredient commonly used in cosmetics.

The cosmetic composition of the present invention may include various bases and/or additives necessary and appropriate for the preparation of formulations thereof, and without departing from the range of reducing the effects thereof, may be prepared by further including known compounds, such as nonionic surfactants, silicone polymers, extenders, fragrances, preservatives, disinfectants, oxidation stabilizers, organic solvents, ionic or nonionic thickeners, softeners, antioxidants, free radical destroyers, opacifiers, stabilizers, emollients, silicone, α-hydroxy acid, anti-foaming agents, moisturizers, vitamins, insect repellents, flavorings, preservatives, surfactants, anti-inflammatory agents, substance P antagonists, fillers, polymers, propellants, alkalinizing or acidifying agents, or coloring agents.

According to one embodiment of the present invention, the cosmetic composition may be any one formulation selected from the group consisting of a solution, a suspension, an emulsion, a paste, a gel, a cream, a lotion, a powder, a soap, a surfactant-containing cleansing, oils, a powder foundation, an emulsion foundations, a wax foundation and a spray.

For example, the cosmetic composition of the present invention may be formulated by, for example, a solution, a suspension, an emulsion, a paste, a gel, a cream, a lotion, a powder, a soap, a surfactant-containing cleansing, oil, a powder foundation, an emulsion foundation, a wax foundation, a spray, and the like, but is not limited thereto. More specifically, the cosmetic composition of the present invention may be formulated as basic cosmetics such as soft lotion, nourishing lotion, lotion, body lotion, nourishing cream, massage cream, moisture cream, hand cream, essence, eye cream, cleansing cream, cleansing foam, cleansing water, pack, gel, patch, oil-in-water (O/W) type and water-in-oil (W/O) type, color cosmetics such as lipstick, makeup base, or foundation, hair fixatives such as hair tonics, gels or mousses, and hair cosmetic compositions such as hair growth agents or hair dyes.

When the formulation of the present invention is the paste, cream, or gel, as a carrier ingredient, animal oils, vegetable oils, wax, paraffin, starch, tragacanth, cellulose derivatives, polyethylene glycol, silicone, bentonite, silica, talc, zinc oxide or the like may be used.

When the formulation of the present invention is the powder or spray, as the carrier ingredient, lactose, talc, silica, aluminum hydroxide, calcium silicate or polyamide powder may be used, and particularly, in the case of the spray, additionally, a propellant such as chlorofluorohydrocarbon, propane/butane or dimethyl ether may be included.

When the formulation of the present invention is the solution or emulsion, a solvent, a solubilizing agent, or an emulsifying agent may be used as a carrier ingredient. For example, water, ethanol, isopropanol, ethyl carbonate, ethyl acetate, benzyl alcohol, benzyl benzoate, propylene glycol, 1,3-butyl glycol oil, glycerol aliphatic esters, polyethylene glycol or sorbitan esters of fatty acids may be used.

When the formulation of the present invention is the suspension, as the carrier ingredient, a liquid diluent such as water, ethanol or propylene glycol, a suspension such as ethoxylated isostearyl alcohol, polyoxyethylene sorbitol ester and polyoxyethylene sorbitan ester, microcrystalline cellulose, aluminum metahydroxide, bentonite, agar, tragacanth, or the like may be used.

When the formulation of the present invention is the surfactant-containing cleansing, as the carrier ingredient, aliphatic alcohol sulfate, aliphatic alcohol ether sulfate, sulfosuccinic acid monoester, isethionate, imidazolinium derivatives, methyltaurate, sarcosinate, fatty acid amide ether sulfate, alkylamido betaine, aliphatic alcohol, fatty acid glyceride, fatty acid diethanolamide, vegetable oil, lanolin derivatives, ethoxylated glycerol esters of fatty acids, or the like may be used.

When the cosmetic composition of the present invention is the soap, the surfactant-containing cleansing formulation, or the surfactant-free cleansing formulation, the composition may be applied to the skin and then wiped off, removed, or washed with water. The soap may be, for example, liquid soap, powdered soap, solid soap, and oil soap, the surfactant-containing cleansing formulation may include cleansing foam, cleansing water, cleansing towels, and cleansing packs, and the surfactant-free cleansing formulation may be cleansing cream, cleansing lotion, cleansing water, and cleansing gel, but are not limited thereto.

According to one embodiment of the present invention, the skin inflammation may be any one disease selected from the group consisting of chronic skin inflammation, allergic skin inflammation, atopic dermatitis, and psoriasis.

According to one embodiment of the present invention, the skin wound may be diabetic foot ulcer or bedsore.

Yet another aspect of the present invention provides a food composition for preventing or improving skin wound or inflammation including an extract of at least one plant selected from the group consisting of Ligustici Rhizoma, Polygala Root, and Longan Arillus, or a compound represented by Chemical Formula 1 or Chemical Formula 2 below or a pharmaceutically acceptable salt thereof:

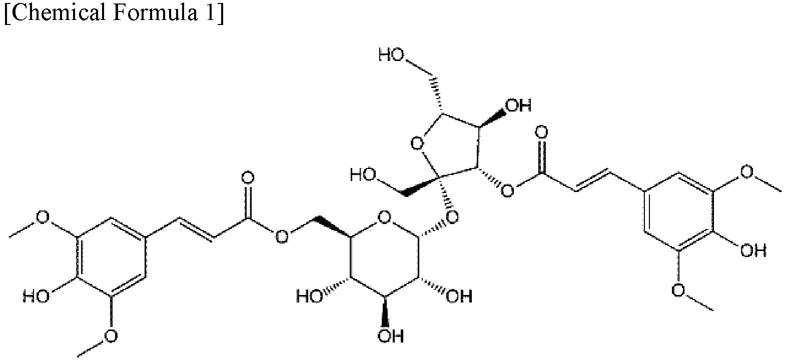

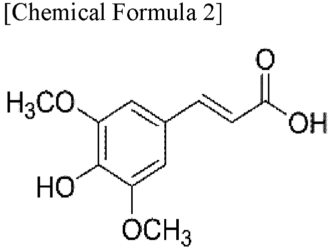

According to one embodiment of the present invention, the extract may be extracted with any one or more solvent selected from the group consisting of water, alcohols having 1 to 4 carbon atoms, and mixed solvents thereof.

The food composition of the present invention may be prepared by adding raw materials and ingredients to be commonly added in the art, and may contain various flavoring agents or natural carbohydrates as additional ingredients like general food compositions, in addition to containing an extract of at least one plant selected from the group consisting of Ligustici Rhizoma, Polygala Root, and Longan Arillus, or a compound represented by Chemical Formula 1 or 2 or a pharmaceutically acceptable salt thereof as an active ingredient.

According to one embodiment of the present invention, the natural carbohydrates may include general sugars, such as monosaccharides (e.g., glucose, fructose, etc.); disaccharides (e.g., maltose, sucrose, etc.); and polysaccharides (e.g., dextrin, cyclodextrin, etc.), and sugar alcohols such as xylitol, sorbitol, and erythritol. The flavoring agents may include natural flavoring agents (tauumatin), stevia extract (e.g., rebaudioside A, glycyrginine, etc.), and/or synthetic flavoring agents (saccharin, aspartame, etc.).

The food composition of the present invention may be formulated as a food composition by further including one or more foodologically acceptable or pharmaceutically acceptable carriers in addition to the active ingredients described above. The formulation form of the food composition may be tablets, capsules, powders, granules, liquids, pills, liquids, syrups, juices, suspensions, emulsions, drops, or the like. For example, for formulation in the form of tablets or capsules, the active ingredient may be combined with an oral non-toxic pharmaceutically acceptable inert carrier, such as ethanol, glycerol, and water.

The food composition of the present invention may include a vitamin mixture consisting of vitamin A acetate, vitamin E, vitamin B 1, vitamin B2, vitamin B6, vitamin B12, vitamin C, biotin, nicotinic acid amide, folic acid, calcium pantothenate, and one or more minerals that may be commonly added in the art, such as ferrous sulfate, zinc oxide, magnesium carbonate, potassium phosphate monobasic, potassium phosphate dibasic, potassium citrate, calcium carbonate, and magnesium chloride.

If necessary, suitable binders, lubricants, disintegrants and coloring agents may also be included as the mixture. The suitable binder may include natural sugar such as starch, gelatin, glucose or beta-lactose, natural and synthetic gums such as corn sweetener, acacia, tragacanth or sodium oleate, sodium stearate, magnesium stearate, sodium benzoate, sodium acetate, sodium chloride, and the like. The disintegrant may include starch, methyl cellulose, agar, bentonite, xanthan gum, etc.

These ingredients may be used independently or in combination, and the ratio of these additives may be selected in the range of 0 to about 20 parts by weight per 100 parts by weight of the food composition of the present invention, but are not limited thereto.

Meanwhile, various foods may be prepared by applying preparation methods of various formulations known to those skilled in the art to the food composition of the present invention. For example, the food composition of the present invention may be prepared in a typical health functional food formulation, such as beverages, pills, and powders, but is not limited thereto.

According to one embodiment of the present invention, the skin inflammation may be any one disease selected from the group consisting of chronic skin inflammation, allergic skin inflammation, atopic dermatitis, and psoriasis.

According to one embodiment of the present invention, the skin wound may be diabetic foot ulcer or bedsore.

Yet another aspect of the present invention provides a method for treating skin wound or inflammation including administering the composition to a subject.

The composition of the present invention is applied to the affected area of the subject by a method such as direct application and thus may be effectively used to treat skin wound or skin inflammation existing on the skin of the subject.

### [Modes for the Invention]

Hereinafter, the present invention will be described in more detail with reference to Examples. However, these Examples are only illustrative the present invention, and the scope of the present invention is not limited to these Examples.

### Preparation Example 1. Preparation of Ligustici Rhizoma extract

20 g of dried Ligustici Rhizoma (Buyoung Pharmaceutical Co., Ltd.) was cleanly washed and cut into small pieces, and then added with 6 times the volume (v/w) of a 20% (v/v) ethanol/water solvent, extracted under reflux for 3 hours at 90 ± 5°C in an earthenware boiling pot and then filtered (10 µm or less). The remaining solid was added with 4 times the volume (v/w) of 20% (v/v) ethanol/water solvent again and then secondarily extracted under the same conditions as above and then filtered (10 µm or less). After extraction, the extract was concentrated under reduced pressure (16 to 21 brix), and then a concentrate was sterilized (80 to 90°C) and freeze-dried.

### Preparation Example 2. Preparation of Polygala Root extract

20 g of dried Polygala Root (Buyoung Pharmaceutical Co., Ltd.) was cleanly washed and cut into small pieces, and then added with 6 times the volume (v/w) of a 20% (v/v) ethanol/water solvent, extracted under reflux for 3 hours at 90 ± 5°C in an earthenware boiling pot and then filtered (10 µm or less). The remaining solid was added with 4 times the volume (v/w) of 20% (v/v) ethanol/water solvent again and then secondarily extracted under the same conditions as above and then filtered (10 µm or less). After extraction, the extract was concentrated under reduced pressure (16 to 21 brix), and then a concentrate was sterilized (80 to 90°C) and freeze-dried.

### Preparation Example 3. Preparation of Longan Arillus extract

20 g of dried Longan Arillus (Buyoung Pharmaceutical Co., Ltd.) was cleanly washed and cut into small pieces, and then added with 6 times the volume (v/w) of a 20% (v/v) ethanol/water solvent, extracted under reflux for 3 hours at 90 ± 5°C in an earthenware boiling pot and then filtered (10 µm or less). The remaining solid was added with 4 times the volume (v/w) of 20% (v/v) ethanol/water solvent again and then secondarily extracted under the same conditions as above and then filtered (10 µm or less). After extraction, the extract was concentrated under reduced pressure (16 to 21 brix), and then a concentrate was sterilized (80 to 90°C) and freeze-dried.

### Preparation Example 4. Preparation of Ligustici Rhizoma and Polygala Root extract

10 g of dried Ligustici Rhizoma (Buyoung Pharmaceutical Co., Ltd.) and 10 g of dried Polygala Root (Buyoung Pharmaceutical Co., Ltd.) were cleanly washed and cut into small pieces, and then added with 6 times the volume (v/w) of a 20% (v/v) ethanol/water solvent, extracted under reflux for 3 hours at 90 ± 5°C in an earthenware boiling pot and then filtered (10 µm or less). The remaining solid was added with 4 times the volume (v/w) of 20% (v/v) ethanol/water solvent again and then secondarily extracted under the same conditions as above and then filtered (10 µm or less). After extraction, the extract was concentrated under reduced pressure (16 to 21 brix), and then a concentrate was sterilized (80 to 90°C) and freeze-dried.

### Preparation Example 5. Preparation of Ligustici Rhizoma and Longan Arillus extract

10 g of dried Ligustici Rhizoma (Buyoung Pharmaceutical Co., Ltd.) and 10 g of dried Longan Arillus (Buyoung Pharmaceutical Co., Ltd.) were cleanly washed and cut into small pieces, and then added with 6 times the volume (v/w) of a 20% (v/v) ethanol/water solvent, extracted under reflux for 3 hours at 90 ± 5°C in an earthenware boiling pot and then filtered (10 µm or less). The remaining solid was added with 4 times the volume (v/w) of 20% (v/v) ethanol/water solvent again and then secondarily extracted under the same conditions as above and then filtered (10 µm or less). After extraction, the extract was concentrated under reduced pressure (16 to 21 brix), and then a concentrate was sterilized (80 to 90°C) and freeze-dried.

### Preparation Example 6. Preparation of Polygala Root and Longan Arillus extract

10 g of dried Polygala Root (Buyoung Pharmaceutical Co., Ltd.) and 10 g of dried Longan Arillus (Buyoung Pharmaceutical Co., Ltd.) were cleanly washed and cut into small pieces, and then added with 6 times the volume (v/w) of a 20% (v/v) ethanol/water solvent, extracted under reflux for 3 hours at 90 ± 5°C in an earthenware boiling pot and then filtered (10 µm or less). The remaining solid was added with 4 times the volume (v/w) of 20% (v/v) ethanol/water solvent again and then secondarily extracted under the same conditions as above and then filtered (10 µm or less). After extraction, the extract was concentrated under reduced pressure (16 to 21 brix), and then a concentrate was sterilized (80 to 90°C) and freeze-dried.

### Experimental Example 1. Preparation for experiments on wound healing effect, anti-inflammatory effect and antibacterial effect

### 1-1. Preparation of cell line

For using Ligustici Rhizoma, Polygala Root and/or Longan Arillus, 3',6-disinapoyl sucrose, or sinapic acid in experiments on wound healing and anti-inflammatory effects, HaCat cells were prepared.

Specifically, the HaCat cells were used after mixing a Dulbecco's Modified Eagle Medium (DMEM) with 10% Fetal Bovine Serum (FBS) and 1% Penicillin/Streptomycin, and incubated in a 75-cm² flask under conditions of 37°C, 95% air, and 5% CO₂. The incubated HaCat cells were refed 2 to 3 times a week, washed with PBS, and then attached cells were isolated with 0.05% Trypsin-0.02% EDTA (Invitrogen^{™}). The isolated cells were centrifuged (5 minutes, 125× g), the accumulated cells were added with the medium, mixed well with a pipette so that the cells were evenly dispersed, and subcultured and used in the experiment.

### 1-2. Configuration of experimental groups

Experimental groups for confirming a wound healing effect (Table 1) and an anti-inflammatory effect (Table 2) of Ligustici Rhizoma, Polygala Root and/or Longan Arillus, 3',6-disinapoyl sucrose, or sinapic acid were configured as follows.

Ligustici Rhizoma, Polygala Root, Longan Arillus, Ligustici Rhizoma + Polygala Root, Ligustici Rhizoma + Longan Arillus, and Polygala Root + Longan Arillus were prepared and used in Preparation Examples 1 to 6, respectively.

**[Table 1]**

| Group | Treating substance | Treating concentration | Cell number (Cells) | Note |
|---|---|---|---|---|
| G1 | - | - | 2.5 × 10^5 cells/well (Migation) | N = 3 |
| G2 | Ligustici Rhizoma | 1 µg/ml | | |
| G3 | Polygala Root | 1 µg/ml | | |
| G4 | Longan Arillus | 1 µg/ml | | |
| G5 | Ligustici Rhizoma + Polygala Root | 1 µg/ml | | |
| G6 | Ligustici Rhizoma + Longan Arillus | 1 µg/ml | | |
| G7 | Polygala Root + Longan Arillus | 1 µg/ml | | |
| G8 | 3'.6-Disinapoyl sucrose | 1 µg/ml | | |
| G9 | Sinapic acid | 1 µg/ml | | |

**[Table 2]**

| Group | Treating substance | Treating concentrati on | Presence or absense of LPS treatment | Cell number (Cells) | Note |
|---|---|---|---|---|---|
| G1 | - | - | - | 5 × 10^5 cells/well | N = 3 |
| G2 | - | - | | | |
| G3 | Ligustici Rhizoma | 1 µg/ml | 10 µg/ml | | |
| G4 | Polygala Root | 1 µg/ml | | | |
| G5 | Longan Arillus | 1 µg/ml | | | |
| G6 | Ligustici Rhizoma + Polygala Root | 1 µg/ml | | | |
| G7 | Ligustici Rhizoma + Longan Arillus | 1 µg/ml | | | |
| G8 | Polygala Root + Longan Arillus | 1 µg/ml | | | |
| G9 | 3'.6-Disinapoyl sucrose | 1 µg/ml | | | |
| G10 | Sinapic acid | 1 µg/ml | | | |

### 1-3. Statistical processing

Parametric multiple comparison procedures were used for the results of the experiments. Assuming normality of the data, the results were tested using parametric one-way ANOVA, and if the results were significant, a post-hoc test was performed using a Dunnett's multiple comparison test to analyze significant differences between the experimental groups.

Statistical analysis was performed using Prism 7.04 (GraphPad Software Inc., San Diego, CA, USA), and if a p value was less than 0.05, it was determined to be statistically significant.

### Experimental Example 2. Confirmation of wound healing effect of Ligustici Rhizoma, Polygala Root and/or Longan Arillus, 3',6-disinapoyl sucrose, or sinapic acid

In order to confirm a wound healing effect of Ligustici Rhizoma, Polygala Root and/or Longan Arillus, 3',6-disinapoyl sucrose, or sinapic acid, cell migration was confirmed by a cell scratch test method.

Specifically, the HaCat cells prepared in Experimental Example 1-1 were seeded in a 6-well plate at a concentration of 2.5 × 10⁵ cells/well using a DMEM complete culture medium and incubated in a 37°C CO₂ incubator for 24 hours. After 24 hours of incubation, the culture medium was replaced with serum free media, and cell scratching was performed using a SPLScar scratcher. Thereafter, the floating cells were washed with 1×PBS and removed, and the medium was replaced with a medium containing a test substance under the same conditions as Table 1 of Experimental Example 1-3 to treat the test substance. Thereafter, the cells were incubated in a CO₂ cell incubator at 37°C, and after 0, 24, and 48 hours of treatment with the test substance, a gap between the cells was measured using a microscope to schematize the extent of a gap between the wounds. Cell migration was calculated using the following Equation.Cell migration = 100 - ((cell interval at each time point/cell interval at 0 time)×100)

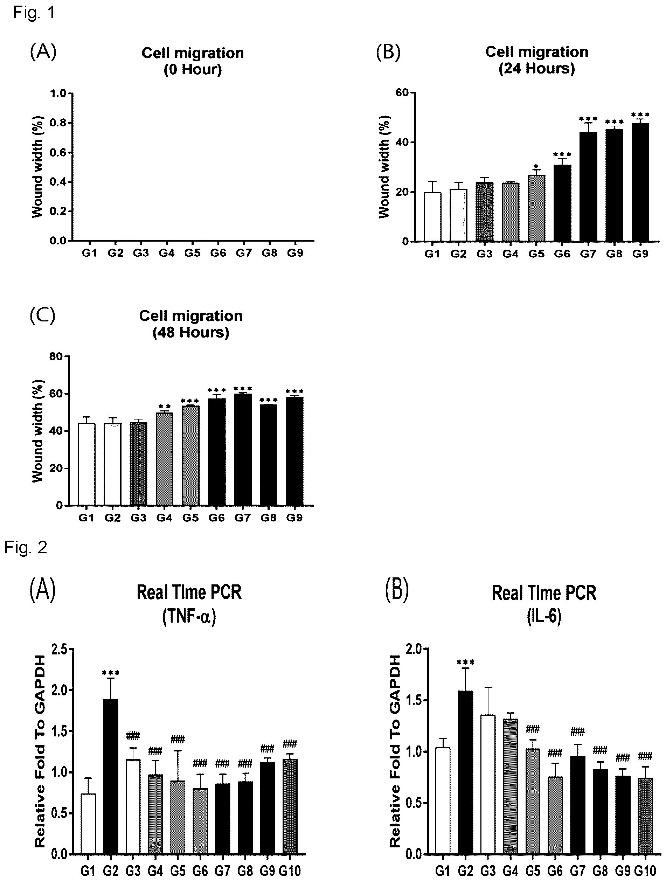

Results of confirming the recovery of cell gaps after 24 hours of administration of test substance (FIG. 1B). It was confirmed that the cell gap recovery rate significantly increased in all test substance general administration groups G5, G6, and G7 compared to a normal control group G1 (p < 0.05 and p < 0.001). In addition, it was confirmed that the cell gap recovery rate was significantly increased in test groups G8 and G9 compared to the normal control group G1. As a result of confirming the statistical significance of the test substance-alone treated group G2 and the general administered group G5 and G6, it was confirmed that the cell gap was significantly increased in the general administered groups G5 and G6 compared to G2 (p < 0.05 and p < 0.01). As a result of confirming the statistical significance of the test substance-alone treated group G3 and the general administered group G5 and G7, it was confirmed that the cell gap was significantly increased in the general administered group G7 compared to G3 (p < 0.001). In addition, as a result of confirming the statistical significance of the test substance-alone treated group G4 and the general administered group G6 and G7, it was confirmed that the cell gap was significantly increased in the general administered group G6 and G7 compared to G4 (p < 0.01 and p < 0.001).

In addition, results of confirming the cell gap recovery after 48 hours of administration of test substance (FIG. 1C). It was confirmed that the cell gap recovery rate significantly increased in G4 among test substance-alone treated groups and all test substance general administration groups G5, G6, and G7 compared to a normal control group G1 (p < 0.01 and p < 0.001). As a result of confirming the statistical significance of the test substance-alone treated group G2 and the general administered groups G5 and G6, it was confirmed that the cell gap was significantly increased in the general administered groups G5 and G6 compared to G2 (p < 0.01). As a result of confirming the statistical significance of the test substance-alone treated group G3 and the general administered groups G5 and G7, it was confirmed that the cell gap was significantly increased in the general administered groups G5 and G7 compared to G3 (p < 0.001). As a result of confirming the statistical significance of the test substance-alone treated group G4 and the general administered groups G6 and G7, it was confirmed that the cell gap was significantly increased in the general administered groups G6 and G7 compared to G4 (p < 0.01 and p < 0.001).

Through these results, as compared to using Ligustici Rhizoma, Polygala Root, and Longan Arillus alone, in the case of using two combined types such as Ligustici Rhizoma and Polygala Root, Polygala Root and Longan Arillus, and Ligustici Rhizoma and Longan Arillus, it was confirmed to exhibit a synergistic effect on wound healing, and it was confirmed that 3',6-disinapoyl sucrose and sinapic acid alone had an excellent effect on wound healing.

### Experimental Example 3. Confirmation of anti-inflammatory effect of Ligustici Rhizoma, Polygala Root and/or Longan Arillus, 3',6-disinapoyl sucrose, or sinapic acid

In order to confirm a wound healing effect of Ligustici Rhizoma, Polygala Root and/or Longan Arillus, 3',6-disinapoyl sucrose, or sinapic acid, the gene expression levels of TNF-α and IL-6 were confirmed by a real time RT-PCR method.

Specifically, the HaCat cells prepared in Experimental Example 1-1 in a 6-well plate were incubated in a CO₂ incubator at 37°C for 24 hours at a concentration of 5 × 10⁵ cells/well using a culture medium of DMEM + 10% fetal bovine serum + 1% Penicillin-Streptomycin. After 24 hours of cell incubation, the cells were treated with LPS (10 µg/ml) for 1 hour using serum free media, and incubated in a CO₂ incubator at 37°C. After 1 hour, the test substance was treated with serum free media under the conditions shown in Table 2 of Experimental Example 1-3 and incubated in a CO₂ incubator at 37°C for 24 hours. After 24 hours, the cell supernatant was removed, and then the cells were washed using phosphate buffer solution (PBS).

RNAwas isolated from the washed cells using Hybrid-R RNA purification KIT. The isolated RNA was quantified using a nanodrop (Take3 Multi-Volume plate, BioTeK, Instruments, VT, USA) and then diluted to the same concentration (100 ng/µl) for each experimental group. Thereafter, the RNA diluted in equal amounts was synthesized into cDNA using PCR, and the gene expression of TNF-α and IL-6 was confirmed through real-time RT-PCR of the synthesized cDNAusing primers in Table 3. Real time RT PCR (Bio-rad, CFX96 touch) conditions to confirm gene expression were as follows: ① Denature reaction 95°C, 2 minutes; ② 95°C 5 seconds; ③ 60°C, 20 seconds + Plate Read; @ GOTO 2, repeat 42 times; ⑥ Melt Curve 65°C to 95°C, increment 0.2°C, 5 seconds + Plate Read; ⑦ End.

**[Table 3]**

| Primer | | Primer sequence (5'→3') | SEQ ID NO: |
|---|---|---|---|
| TNF-α | forward | GCCCAGGCAGTCAGATCATCT | SEQ ID NO: 1 |
| | reverse | TTGAGGGTTTGCTACAACATGG | SEQ ID NO: 2 |
| IL-6 | forward | AATTCGGTACATCCTCGACGG | SEQ ID NO: 3 |
| | reverse | GGTTGTTTTCTGCCAGTGCC | SEQ ID NO: 4 |

As a result, as the result of confirming the gene expression of TNF-α after 24 hours of test substance administration (FIG. 2A), it was confirmed that the gene expression of TNF-α was significantly increased in the normal control group G1 compared to an induced control group G2, and in all test substance administered groups compared to the induced control group G2, the gene expression of TNF-α was significantly decreased (p < 0.001). As a result of confirming the statistical significance of the test substance-alone treated group G3 and the general administered groups G6 and G7, it was confirmed that the gene expression of TNF-α was significantly decreased in the general administered groups G6 and G7 compared to G3 (p < 0.05). As a result of confirming the statistical significance of the test substance-alone treated group G4 and the general administered groups G6 and G8, it was confirmed that the gene expression of TNF-α was significantly decreased in the general administered groups G6 and G8 compared to G4 (p < 0.01 and p < 0.001). As a result of confirming the statistical significance of the test substance-alone treated group G5 and the general administered groups G7 and G8, it was confirmed that the gene expression of TNF-α was significantly decreased in the general administered group G8 compared to G5 (p < 0.05).

In addition, as a result of confirming the gene expression of IL-6 after 24 hours of test substance administration (FIG. 2B), it was confirmed that the gene expression of IL-6 was significantly increased in the normal control group G1 compared to the induced control group G2, and the gene expression of IL-6 was significantly decreased in the test substance administered groups G5, G6, G7, G8, G9 and G10 compared to the induced control group G2 (p < 0.001). As a result of confirming the statistical significance of the test substance-alone treated group G3 and the general administered groups G6 and G7, it was confirmed that the gene expression of IL-6 was significantly decreased in the general administered groups G6 and G7 compared to G3 (p < 0.05).

Through these results, as compared of using Ligustici Rhizoma, Polygala Root, and Longan Arillus alone, in the case of using two combined types such as Ligustici Rhizoma and Polygala Root, Polygala Root and Longan Arillus, and Ligustici Rhizoma and Longan Arillus, it was confirmed to exhibit a synergistic effect on the anti-inflammatory effect, and it was confirmed that 3',6-disinapoyl sucrose and sinapic acid alone had an excellent anti-inflammatory effect.

### Preparation Example 1. Cream composition (1)

An oil phase and an aqueous phase were heated and mixed at 75°C, respectively, and then cooled to room temperature.

**[Table 4]**

| No. | Name of raw material | Part by weight |
|---|---|---|
| 1 | Cetostearyl alcohol | 1.0 |
| 2 | Self-emulsifying glyceryl monostearate | 1.0 |
| 3 | Lipophilic glyceryl monostearate | 2.5 |
| 4 | Micro stalin lead | 2.0 |
| 5 | Methyl paraoxybenzoate | 0.2 |
| 6 | Propyl paraoxybenzoate | 0.1 |
| 7 | Beeswax | 2.0 |
| 8 | Polyethylene glycol monostearate | 2.0 |
| 9 | Sorbitan monostearate | 1.0 |
| 10 | Caprylic/Capric triglyceride | 5.0 |
| 11 | Liquid linoline | 5.0 |
| 12 | Octyldodecyl myristate | 8.0 |
| 13 | Squalane | 8.0 |
| 14 | Concentrated glycerin | 5.0 |
| 15 | 1,3-butylene glycol | 5.0 |
| 16 | Allantoin | 0.1 |
| 17 | Extract of at least one plant selected from the group consisting of Ligustici Rhizoma, Polygala Root, and Longan Arillus, or compound represented by Chemical Formula 1 or Chemical Formula 2 below or pharmaceutically acceptable salt thereof | 10.0 |
| 18 | Flavoring | Trace |
| 19 | Yellow No. 4 | Trace |
| 20 | Purified water | Residual |

### Preparation Example 2. Cream composition (2)

An oil phase and an aqueous phase were heated, dissolved, and mixed at 75°C, respectively, and then cooled to room temperature.

**[Table 5]**

| No. | Name of raw material | Part by weight |
|---|---|---|
| 1 | Cetostearyl alcohol | 2.0 |
| 2 | Lipophilic glyceryl monostearate | 2.5 |
| 3 | Self-emulsifying glyceryl monostearate | 1.5 |
| 4 | Methyl paraoxybenzoate | 0.2 |
| 5 | Propyl paraoxybenzoate | 0.1 |
| 6 | Vaseline | 3.5 |
| 7 | Polyethylene glycol monostearate | 3.5 |
| 8 | Sorbitan sesquioleate | 1.8 |
| 9 | Liquid paraffin | 35.0 |
| 10 | Caprylic/Capric triglyceride | 3.0 |
| 11 | Octyldodecanol | 5.0 |
| 12 | Squalene | 2.0 |
| 13 | Concentrated glycerin | 5.0 |
| 14 | Extract of at least one plant selected from the group consisting of Ligustici Rhizoma, Polygala Root, and Longan Arillus, or compound represented by Chemical Formula 1 or Chemical Formula 2 below or pharmaceutically acceptable salt thereof | 5.0 |
| 15 | Hyaluronic acid extract | 1.0 |
| 16 | Flavoring | Trace |
| 17 | Purified water | Residual |

### Preparation Example 3. Lotion composition

An oil phase and an aqueous phase were heated, mixed, and emulsified at 75°C, respectively, and then cooled to room temperature.

**[Table 6]**

| No. | Name of raw material | Part by weight |
|---|---|---|
| 1 | Cetostearyl alcohol | 2.0 |
| 2 | Stearate | 1.0 |
| 3 | Lipophilic glyceryl monostearate | 1.0 |
| 4 | Self-emulsifying glyceryl monostearate | 2.0 |
| 5 | Vaseline | 1.0 |
| 6 | Polyethylene glycol monostearate | 1.5 |
| 7 | Sorbitan sesquioleate | 1.0 |
| 8 | Liquid paraffin | 5.0 |
| 9 | Squalene | 5.0 |
| 10 | Propyl paraoxybenzoate | 0.2 |
| 11 | Methyl paraoxybenzoate | 0.2 |
| 12 | Concentrated glycerin | 5.0 |
| 13 | Extract of at least one plant selected from the group consisting of Ligustici Rhizoma, Polygala Root, and Longan Arillus, or compound represented by Chemical Formula 1 or Chemical Formula 2 below or pharmaceutically acceptable salt thereof | 15.0 |
| 14 | Triethanol amine | 0.5 |
| 15 | Carboxyvinyl polymer | 0.2 |
| 16 | Flavoring | Trace |
| 17 | Purified water | Residual |

### Preparation Example 4. Skin lotion composition

An aqueous phase and an ethanol phase were prepared, mixed, and then filtered, respectively.

**[Table 7]**

| No. | Name of raw material | Part by weight |
|---|---|---|
| 1 | Glycerin | 2.0 |
| 2 | 1,3-butylene glycol | 2.0 |
| 3 | Citric acid | 0.01 |
| 4 | Ethanol | 15.0 |
| 5 | Polyoxyethylene hydrogenated castor oil | 1.0 |
| 6 | Extract of at least one plant selected from the group consisting of Ligustici Rhizoma, Polygala Root, and Longan Arillus, or compound represented by Chemical Formula 1 or Chemical Formula 2 below or pharmaceutically acceptable salt thereof | 25.0 |
| 7 | Flavorings | Trace |
| 8 | Witch Hazel Extract | 1.0 |
| 9 | Purified water | Residual |

### Preparation Example 5. Essence composition

An aqueous phase and an ethanol phase were prepared, mixed, and then filtered, respectively.

**[Table 8]**

| No. | Name of raw material | Part by weight |
|---|---|---|
| 1 | Concentrated glycerin | 15.0 |
| 2 | 1,3-butylene glycol | 5.0 |
| 3 | Allantoin | 0.1 |
| 4 | Ethanol | 7.0 |
| 5 | Methyl paraoxybenzoate | 0.15 |
| 6 | Triethanol amine | 0.15 |
| 7 | Polyoxyethylene hydrogenated castor oil | 1.0 |
| 8 | Tocopherol acetate | 0.5 |
| 9 | Carboxyvinyl polymer | 0.15 |
| 10 | Extract of at least one plant selected from the group consisting of Ligustici Rhizoma, Polygala Root, and Longan Arillus, or compound represented by Chemical Formula 1 or Chemical Formula 2 below or pharmaceutically acceptable salt thereof | 30.0 |
| 11 | Flavoring | Trace |
| 12 | Purified water | Residual |

### Preparation Example 6. Pack composition

An aqueous phase and an ethanol phase were dispersed, dissolved, mixed, and then cooled to room temperature, respectively.

**[Table 9]**

| No. | Name of raw material | Part by weight |
|---|---|---|
| 1 | Polyvinyl alcohol | 15.0 |
| 2 | Sodium Carboxymethylcellulose | 0.3 |
| 3 | Concentrated glycerin | 3.0 |
| 4 | Allantoin | 0.1 |
| 5 | Disodium ethylenediaminetetraacetate | 0.01 |
| 6 | Polyethylene glycol | 1.0 |
| 7 | Ethanol | 6.0 |
| 8 | Methyl paraoxybenzoate | 0.15 |
| 9 | Propyl paraoxybenzoate | 0.05 |
| 10 | DL-panthenol | 0.1 |
| 11 | Polyoxyethylene (12) nonylphenyl ether | 0.5 |
| 12 | Extract of at least one plant selected from the group consisting of Ligustici Rhizoma, Polygala Root, and Longan Arillus, or compound represented by Chemical Formula 1 or Chemical Formula 2 below or pharmaceutically acceptable salt thereof | 45.0 |
| 13 | Flavoring | Trace |
| 14 | Purified water | Residual |

The present invention has been described above with reference to preferred embodiments thereof. It will be understood to those skilled in the art that the present invention may be implemented as a modified form without departing from an essential characteristic of the present invention. Therefore, the disclosed exemplary embodiments should be considered in an illustrative viewpoint rather than a restrictive viewpoint. The scope of the present invention is defined by the appended claims rather than by the foregoing description, and all differences within the scope of equivalents thereof should be construed as being included in the present invention.

## Claims

1. A pharmaceutical composition for preventing or treating skin wound or inflammation comprising an extract of at least one plant selected from the group consisting of Ligustici Rhizoma, Polygala Root, and Longan Arillus, or a compound represented by Chemical Formula 1 or Chemical Formula 2 below or a pharmaceutically acceptable salt thereof:

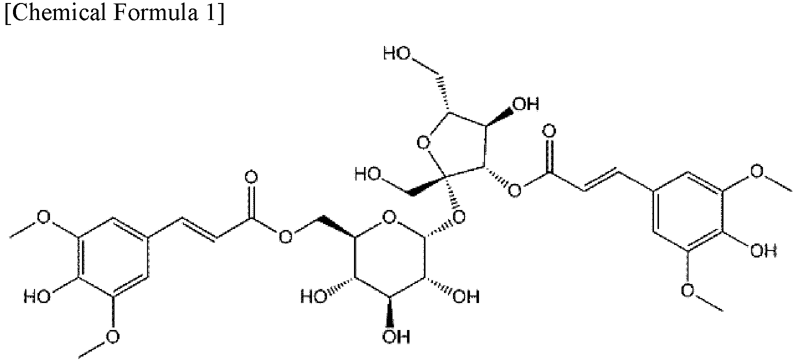

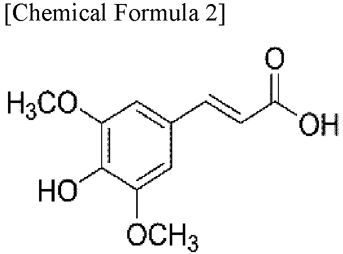

2. The pharmaceutical composition for preventing or treating skin wound or inflammation of claim 1, wherein the extract is extracted with any one solvent selected from the group consisting of water, alcohols having 1 to 4 carbon atoms, and mixed solvents thereof.

3. The pharmaceutical composition for preventing or treating skin wound or inflammation of claim 1, wherein the pharmaceutical composition is at least one formulation selected from the group consisting of creams, gels, patches, sprays, emulsions, ointments, emplastrum, lotions, liniment agents, paste agents, solvents, suspensions, packs, patches and cataplasma.

4. The pharmaceutical composition for preventing or treating skin wound or inflammation of claim 1, wherein the inflammation is skin inflammation or arthritis.

5. The pharmaceutical composition for preventing or treating skin wound or inflammation of claim 4, wherein the skin inflammation is any one disease selected from the group consisting of chronic skin inflammation, allergic skin inflammation, atopic dermatitis, and psoriasis.

6. The pharmaceutical composition for preventing or treating skin wound or inflammation of claim 1, wherein the skin wound is diabetic foot ulcer or bedsore.

7. A cosmetic composition for preventing or improving skin wound or inflammation comprising an extract of at least one plant selected from the group consisting of Ligustici Rhizoma, Polygala Root, and Longan Arillus, or a compound represented by Chemical Formula 1 or Chemical Formula 2 below or a pharmaceutically acceptable salt thereof:

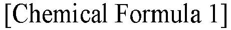

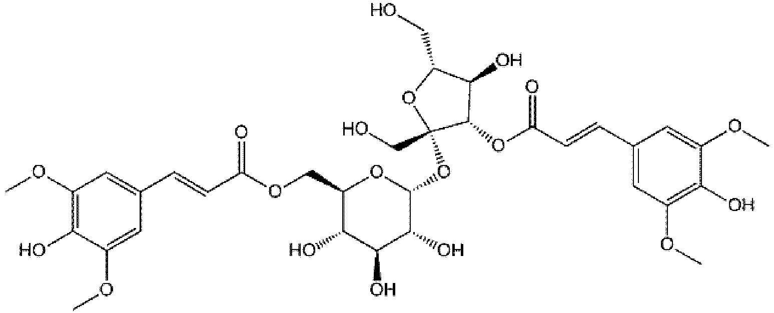

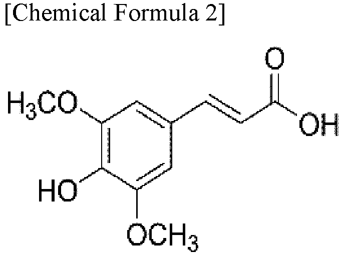

8. The cosmetic composition for preventing or improving skin wound or inflammation of claim 7, wherein the extract is extracted with any one solvent selected from the group consisting of water, alcohols having 1 to 4 carbon atoms, and mixed solvents thereof.

9. The cosmetic composition for preventing or improving skin wound or inflammation of claim 7, wherein the cosmetic composition is any one formulation selected from the group consisting of a solution, a suspension, an emulsion, a paste, a gel, a cream, a lotion, a powder, a soap, a surfactant-containing cleansing, oils, a powder foundation, an emulsion foundations, a wax foundation and a spray.

10. The cosmetic composition for preventing or improving skin wound or inflammation of claim 7, wherein the skin inflammation is any one disease selected from the group consisting of chronic skin inflammation, allergic skin inflammation, atopic dermatitis, and psoriasis.

11. The cosmetic composition for preventing or improving skin wound or inflammation of claim 7, wherein the skin wound is diabetic foot ulcer or bedsore.

12. A food composition for preventing or improving skin wound or inflammation comprising an extract of at least one plant selected from the group consisting of Ligustici Rhizoma, Polygala Root, and Longan Arillus, or a compound represented by Chemical Formula 1 or Chemical Formula 2 below or a pharmaceutically acceptable salt thereof:

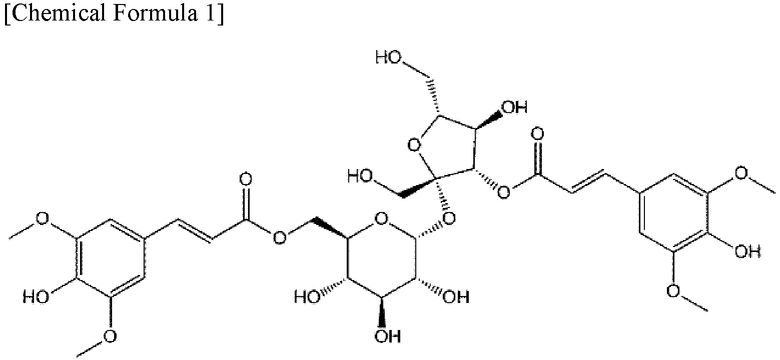

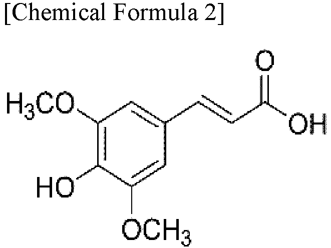

13. The food composition for preventing or improving skin wound or inflammation of claim 12, wherein the extract is extracted with any one solvent selected from the group consisting of water, alcohols having 1 to 4 carbon atoms, and mixed solvents thereof.

14. The food composition for preventing or improving skin wound or inflammation of claim 12, wherein the skin inflammation is any one disease selected from the group consisting of chronic skin inflammation, allergic skin inflammation, atopic dermatitis, and psoriasis.

15. The food composition for preventing or improving skin wound or inflammation of claim 12, wherein the skin wound is diabetic foot ulcer or bedsore.

16. A method for treating skin wound or inflammation comprising administering the composition of claim 1 to a subject.
